# EUROPEAN PATENT APPLICATION

(11) **EP 1 357 194 A2**
(43) Date of publication of application: **29.10.2003**
(21) Application number: 03252578.4
(22) Date of filing: 24.04.2003
(51) Int. Cl.: C12Q 1/00, G01N 27/28, G01N 27/30

(54) **Adapter for the connection of a biosensor to a measuring device**

(30) Priority: 26.04.2002 JP 2002126810
(71) Applicant: MATSUSHITA ELECTRIC INDUSTRIAL CO., LTD., Kadoma-shi, Osaka 571-8501 (JP)
(72) Inventor: Taniike, Yuko, Osaka-shi, Osaka 542-0066 (JP); Ikeda, Shin, Katano-shi, Osaka 576-0022 (JP); Yoshioka, Toshihiko, Hirakata-shi, Osaka 573-0035 (JP)
(74) Representative: Bradley, Josephine Mary

(57) **Abstract**

A face-type biosensor capable of being easily connected to a measuring device through an adapter. The biosensor includes a working electrode lead and a counter electrode lead that face each other. The biosensor is inserted into said measuring device through the adapter (2). A pressing portion in the adapter brings the second counter electrode lead into contact with first counter electrode lead.

## Description

### Field of the Invention

The present invention relates to a biosensor, adaptor and measuring device for determining, with high speed and high accuracy, the quantity of a substrate contained in a sample. Methods for quantitative analysis of saccharides such as sucrose and glucose have been developed. These methods include a polarimeter method, colorimetric method, reduction titrimetrical method, and methods using various kinds of chromatography. However, these methods have poor accuracy because they are not capable of determining with accuracy the quantity of saccharides in a sample. For example, the polarimeter method involves a simple and easy method of operation. However, the accuracy of the method is highly affected by the temperature at the time of operation. Therefore, the polarimeter method is not appropriate as the method for simple and easy means for determining of the quantity of saccarides in home by ordinary persons.

In recent years, there have been developed biosensors of various types making use of the specific catalytic activity of an enzyme. For example, it is known to use glucose oxidase (EC1.1.3.4), hereinafter abbreviated as GOD, as an enzyme and an oxygen electrode or a hydrogen peroxide electrode for the electrochemical determination of the quantity of glucose in a sample. GOD selectively oxidizes β-D-glucose as a substrate into D-glucono-δ-lactone with oxygen as an electron mediator. In the presence of oxygen, oxygen is reduced to hydrogen peroxide in the oxidation process by GOD. The amount of oxygen decreased is measured with an oxygen electrode, or the amount of hydrogen peroxide increased is measured with a hydrogen peroxide electrode. Since the amount of oxygen decreased or the amount of hydrogen peroxide increased is in proportion to the amount of glucose contained in a sample, the determination of glucose is achieved from the amount of oxygen decreased or the amount of hydrogen peroxide increased.

In the above method, the quantity of glucose in a sample can be determined with high accuracy by making use of the specificity of the enzyme reaction. However, as assumed from the reaction process, the results of the measurement have a disadvantage in that they are highly affected by the concentration of oxygen contained in the sample. If a sample contains no oxygen, the measurement becomes impossible.

Thus, there have been developed new types of glucose sensors using no oxygen as an electron mediator, but using an organic compound or a metal complex such as potassium ferricyanide, a ferrocene derivative, or a quinone derivative as the electron mediator. These sensors have a working electrode and a counter electrode. In the sensor of this type, a reductant of the electron mediator generated as a result of the enzyme reaction is oxidized on the working electrode and the concentration of glucose contained in a sample can be obtained from the quantity of the oxidation current. On the counter electrode, an oxidant of the electron mediator is reduced and the reaction of producing a reductant of the electron mediator proceeds. The use of such an organic compound or a metal complex as an electron mediator instead of oxygen makes it possible to form a reagent layer by carrying a prescribed amount of GOD and the electron mediators in a stable condition correctly on the electrode, so that the determination of glucose can be achieved with high accuracy without being affected by the concentration of oxygen in the sample. Since the reagent layer containing an enzyme and an electron mediator can also be integrated with the electrode system in the nearly dry state, a disposable type glucose sensor based on this technology has attracted much attention in recent years. A typical example thereof is a biosensor disclosed in Japanese Patent Laid-Open Publication No. Hei. 3-202764. In the disposable type glucose sensor, the sensor is detachably connected to a measuring device. The glucose concentration can easily be measured with the measuring device by introducing a sample into the sensor.

In the measurement using the above glucose sensor, the concentration of a substrate in a sample can easily be determined with a sample amount on the order of several microliters. However, in recent years, there has been interest in the development of a biosensor capable of measuring a minute amount (1 µl or lower) of a sample. The conventional electrochemical glucose sensors are flat-type biosensors in which an electrode system is provided on a single plane. When measuring a sample in a very minute amount, an increase in the resistance to the charge transfer (mainly ion transfer) between the electrodes may lead to a decrease in measuring sensitivity or a variation in the results of measurement.

A face-type biosensor has been proposed in which a working electrode and a counter electrode are provided at positions facing each other. The quantity of a substrate such as glucose contained in a sample can be determined in the face-type sensor with high accuracy and high sensitivity, as compared with the conventional flat-type biosensors. For example, because the arrangement of the working electrode and the counter electrode are at positions facing each other, a smooth ion transfer between the working electrode and the counter electrode is possible.

However, when the face-type sensor is electrically connected to a measuring device, the shape of the leads for the connecting terminals of the measuring device must be different from the shape of the leads in the face-type biosensor because the working electrode and the counter electrode are not on the same plane. For example, Japanese Patent Laid-open Publication No. Hei 11-352093 discloses a face-type biosensor comprising a working electrode base plate and a counter electrode base plate with through-holes to expose a working electrode lead and a counter electrode lead. The leads are in a front-and-back reversed direction, i.e., the working electrode lead faces the counter electrode base plate while the counter electrode lead faces the working electrode base plate. Japanese Patent Laid-open Publication No. Hei 9-159642 discloses a face-type biosensor having a similar front-and-back reversed direction arrangement, except that cut portions, and not through holes, are formed in the working electrode base plate and the counter electrode base plate. In these biosensors, because the working electrode lead and the counter electrode lead are exposed in a front-and-back reversed direction, there is a need for the connecting terminals for the working electrode lead and the counter electrode lead on the measuring device side to have special shapes different from those of the flat-type biosensor where the leads are not in a front-and-back reversed direction.

Japanese Patent Laid-open Publication No. Hei 11-125618 discloses a face-type biosensor comprising a longer lower base plate and a shorter upper base plate. The end portion of the lower base plate does not overlap with the upper base plate. The lead portions of a working electrode and a counter electrode are formed on the inner side of the longer lower base plate. The working electrode and the counter electrode are formed on a shorter upper base plate. An adhesive layer or a spacer is interposed between the plates. The working electrode and the counter electrode electrically communicate with their respective lead portions through the adhesive layer or the spacer. Again, the shape of connecting terminals on the measuring device side may be the same as the flat-type biosensor, but they require the formation of leads on the sensor side so that these leads penetrate through the adhesive layer or the spacer, the base plates, and the like, thereby making the production process cumbersome and complicated.

As described above, in the conventional face-type biosensor, it is required to make the shape of the lead of the sensor or the connecting terminal of the measuring device a special shape, which is different from that of the flat-type biosensor.

Accordingly, it is an object of the present invention to provide a face-type biosensor which has simple leads and can easily be connected to a measuring device.

### Brief Summary of the Invention

The present invention relates to a biosensor comprising a first base plate, a second base plate, a first electrode, a second electrode, a first lead electrically connected to the first electrode, a second lead, a third lead electrically connected to the second electrode, a spacer member provided between the first base plate and the second base plate to form a sample feed portion, and a reagent containing at least one enzyme, which reagent is provided so that a part of the reagent is exposed to the sample feed portion.

The invention is characterized in that the first electrode, the first lead, and the second lead are provided on the first base plate and on the side facing the second base plate; the second electrode and the third lead arc provided on the second base plate and on the side facing the first base plate. Either the first electrode or the second electrode can function as a working electrode while the other electrode functions as a counter electrode; and the second lead and the third lead are brought into electrical contact with each other by pressing the first base plate and/or the second base plate toward the other base plate.

It is preferable that the third lead is divided into more than one portion and that the biosensor includes more than one second lead.

The present invention further relates to an adapter comprising a sensor slot into which a part of the above-mentioned biosensor is inserted, a pressing portion for pressing a part of the second base plate of the biosensor to bring the second lead and the third lead into electrical contact with each other, and a connection port for connecting the biosensor with a measuring device.

It is preferable that a part of the pressing portion comprises an elastic material.

The present invention further relates to a measuring device which is detachably connected to the above-mentioned biosensor through the above-mentioned adapter, and which measures a substrate contained in a sample solution that is fed to the sample feed portion of the biosensor. The measuring device comprises a first terminal electrically connected to the first lead of the biosensor, a second terminal electrically connected respectively to the second lead of the biosensor, a means for applying a voltage between the first electrode and the second electrode of the biosensor through the first terminal and the second terminal, and a means for measuring an electrical change between the first electrode and the second electrode.

It is preferable that the above-mentioned measuring device includes a plurality of second terminals.

Furthermore, it is preferable that the above-mentioned measuring device comprises a means for measuring an electrical change between the first terminal and the plurality of second terminals, a means for sensing a second terminal electrochemically connected to the first terminal out of the plurality of second terminals, a means for determining a type of biosensor on the basis of the number and the position of the sensed second terminal, and a means for correcting a measurement result according to the type of biosensor.

The present invention further relates to a measuring device which is detachably connected to the above-mentioned biosensor and which measures a substrate contained in a sample solution to be fed to the sample feed portion of the biosensor, characterized in that it comprises a first terminal electrically connected to the first lead of the biosensor, a second terminal electrically connected to the second lead of the biosensor, a means for applying a voltage between the first electrode and the second electrode of the biosensor, a means for measuring an electrical change between the first electrode and the second electrode, a sensor port into which at least a part of the biosensor is inserted, and a pressing portion for pressing a part of the second base plate of the biosensor to bring the second lead and the third lead into electrical contact with each other.

It is preferable that the above-mentioned measuring devicc comprises a plurality of second terminals.

It is preferable that the above-mentioned measuring device includes a means for measuring an electrical change between the first terminal and the plurality of the second terminals, a means for sensing a second terminal electrochemically connected to the first terminal, a means for determining a type of biosensor on the basis of a number and a position of the sensed second terminal, and a means for correcting a measurement result according to the type of biosensor.

It is preferable that a part of the pressing portion comprises an elastic material.

It is preferable that the pressing portion is movable so that a position to be pressed of the second base plate of the biosensor is determined by the position of the pressing portion.

It is preferable that the second terminal serves also as the pressing portion.

While the novel features of the invention are set forth particularly in the appended claims, the invention, both as to organization and content, will be better understood and appreciated, along with other objects and features thereof, from the following detailed description taken in conjunction with the drawings.

### Brief Description of the Several Views of the Drawings

FIG. 1 is a schematic view showing the constitution of a biosensor, an adapter, and a measuring device, in accordance with a first embodiment of the present invention.
FIG. 2(a) is an exploded perspective view of the biosensor of the first embodiment of the invention, except for the reagent layer and the surfactant layer.
FIG. 2(b) is a longitudinal sectional view of the first embodiment of the invention showing separately the sensor slot of the measuring device, the adapter, and the biosensor.
FIG. 2(c) is a longitudinal sectional view showing the sensor slot of the measuring device, the adapter, and the biosensor of the first embodiment of the invention connected to one another.
FIG.3 is a front view of the adapter of the first embodiment of the invention seen from the direction of the sensor port.
FIG.4 is a schematic view showing the constitution of a biosensor, an adapter, and a measuring device in the second embodiment of the present invention.
FIG. 5(a) an exploded perspective view of the biosensor of the second embodiment of the invention, except for the reagent layer and the surfactant layer of the biosensor.
FIG. 5(b) is a longitudinal sectional view of the second embodiment of the invention showing separately the sensor slot of the measuring device, the adapter, and the biosensor.
FIG. 5(c) is a longitudinal sectional view of the second embodiment of the invention showing the sensor slot of the measuring device, the adapter, and the biosensor connected to one another.
FIG. 6(a) is a front view of an adapter of the second embodiment of the invention with pressing portion as seen from the direction of the sensor port of the adapter.
FIG. 6(b) is another front view of an adapter of the second embodiment with pressing portion, seen from the direction of the sensor port of the adapter.
FIG. 6(c) is further another front view of an adapter of the second embodiment of the invention with pressing portion as seen from the direction of the sensor port of the adapter.
FIG. 7(a) is a schematic view showing the measuring device when the pressing portion is closed.
FIG. 7(b) is a schematic view showing the measuring device when the pressing portion is open.
FIG. 7(c) is a schematic view showing the measuring device when the biosensor is connected to the measuring device.

### Detailed Description of the Invention

In one embodiment, the biosensor of the present invention comprises a first base plate, a second base plate, a first electrode, a second electrode, a first lead electrically connected to the first electrode, a second lead, a third lead electrically connected to the second electrode, a spacer member provided between the first base plate and the second base plate to form a sample feed portion, and a reagent containing at least an enzyme. A part of the reagent is exposed to the sample feed portion and is characterized in that the first electrode, the first lead, and the second lead are provided on the first base plate and on the side facing the second base plate. The second electrode and the third lead are provided on the second base plate and on the side facing the first base plate. Either the first electrode or the second electrode can function as a working electrode while the other electrode functions as a counter electrode. The second lead and the third lead are brought into electrical contact with each other by pressing the first base plate and/or the second base plate toward the other base plate. Where the second and third leads are on the second base plate, the leads are brought into electrical contact with each other such that at least the end portion of the first lead and at least the end portion of the second lead are exposed to the outside by pressing the first base plate and/or the second base plate toward the other base plate. Thus, the second lead and the third lead are brought into electrical contact with each other by pressing the first base plate and/or the second base plate toward the other base plate, making possible the electrical connection to the connecting terminal on the measuring device side using the end portions of the first lead and the second lead provided on the same face of the first base plate. Thus, the biosensor having this configuration can be used in a measuring device for conventional flat-type biosensors. In addition, since there is no need to form through holes or cut portions to allow the leads to electrically communicate, the sensors have a simpler design which simplifies the process for producing the biosensors.

It is preferable that the third lead is divided into more than one portion and that the sensor comprises more than one second lead. Thus, if there is more than one second lead, the second lead brought into contact with the third lead may be changed corresponding to the production lot of a sensor, so that the determination of the production lot of the sensor can be made in a measuring device by detecting which second lead is connected to the third lead when the sensor inserted into the measuring device. Therefore, labor costs can be saved because it is not necessary to input by hand the lot number into a measuring device before measurement or to insert a separate chip in the measuring device for determining the lot number before the measurement.

The adapter of the present invention comprises a sensor slot into which a part of the above biosensor is inserted. The adapter further includes a pressing portion for pressing a part of the second base plate of the biosensor inserted to bring the second lead and the third lead into electrical contact with each other, and a connection port for connecting with a measuring device. Thus, the second base plate of the sensor is pressed by inserting the biosensor of the present invention into the sensor slot of the adapter, so that the second lead and the third lead of the sensor can easily be brought into contact with each other.

It is preferable that at least a part of the pressing portion comprises an elastic material. Thus, it is possible to press the biosensor securely into the adapter and to bring the second lead and the third lead securely into contact with each other.

The measuring device in a preferred embodiment of the present invention is a measuring device in which the above third lead is divided into more than one portion and is detachably connected through the above adapter to a biosensor having more than one second lead. The measuring device measures a substrate contained in a sample solution fed to the sample feed portion of the biosensor. The biosensor comprises a first terminal electrically connected to the first lead of the biosensor, more than one second terminal electrically connected respectively to more than one second lead of the biosensor, a means for applying a voltage between the first electrode and the second electrode of the biosensor through the first terminal and more than one second terminal, and a means for measuring an electrical change between the first electrode and the second electrode. The determination of the production lot of the biosensor can be made in the measuring device by using an adapter with the pressing portion formed at different positions corresponding to the production lot of the sensor and connecting the biosensor to the measuring device through the adaptor. The third lead of the biosensor is divided into more than one lead. The biosensor further comprises more than one second lead. The measuring device detects what second lead is connected to the third counter electrode lead by using more than one second terminal on the measuring device side. Therefore, labor costs can be saved because it is not necessary to input by hand of the lot number into a measuring device before measurement or to insert a chip into the measuring device determining the lot number before measurement.

The measuring device in a second preferred embodiment of the present invention is a measuring device that is detachably connected to the above biosensor and which measures a substrate contained in a sample solution fed to the sample feed portion of the biosensor. The measuring device comprises a first terminal electrically connected to the first lead of the biosensor, a second terminal electrically connected to the second lead of the biosensor, a means for applying a voltage between the first electrode and the second electrode of the biosensor through the first terminal and the second terminal, a means for measuring an electrical change between the first electrode and the second electrode, a sensor port for inserting at least a part of the biosensor, and a pressing portion for pressing at least a part of the second base plate of the biosensor inserted to bring the second lead and the third lead into electrical contact with each other. Thus, the substrate contained in the sample solution can easily be measured using the biosensor of the present invention without an adapter.

It is preferable that a part of the pressing portion comprises an elastic material. Thus, it is possible to press the biosensor securely into the adaptor to bring the second lead and the third lead securely into contact with each other.

Furthermore, it is preferable that the pressing portion be movable. Thus, it is possible to press the biosensor securely into the adaptor and to easily measure the substrate contained in the sample solution.

Furthermore, it is preferable to make the second terminal serve also as the pressing portion. Thus, the number of components comprising measuring device can be reduced so as to simplify the the measuring device.

In the present invention, the first base plate can be made of any material provided it is a material having electrically insulating properties and stiffness sufficient for storage and measurement. The second base plate can be made of the same material as the first base plate, but it must have flexibility such that the second base plate can be bent by a force from the direction perpendicular to the base plate surface to bring the second lead, which is formed on the first base plate, and the third lead, which is formed on the second base plate, into contact with each other. Also, the second base plate must have strength such that the second base plate can endure the stress imposed in the direction perpendicular to the base plate surface when bent. The material for the first base plate and the second base plate may include, for example, thermoplastic resins such as polyethylene, polystyrene, polyvinyl chloride, polyamide, and saturated polyester resins; and thermosetting resins such as urea resins, melamine resins, phenol resins, epoxy resins, and unsaturated polyester resins. In particular, polyethylene terephtbalate is preferred from the viewpoint of electrode adhesion. It is possible to use a spring or the like as the elastic member.

The present invention will hereinafter be further illustrated by the drawings. The following embodiments will describe, as an example, a biosensor used for the determination of glucose, an adapter, a measuring device, and a determination method. However, the substrate is not limited to glucose. Also, the invention is not limited to a biosensor wherein the first electrode is a working electrode and the second electrode is a counter electrode, and the first base plate, the second base plate, the first lead, the second lead, the third lead, the first terminal, and the second terminal are a working electrode base plate, a counter electrode base plate, a counter electrode lead, a first counter electrode lead, a second counter electrode lead, a working electrode terminal, and a counter electrode terminal, respectively. The first electrode may be a counter electrode and the second electrode may be a working electrode, and the first base plate, the second base plate, the first lead, the second lead, the third lead, the first terminal, and the second terminal may be a counter electrode base plate, a working electrode base plate, a counter electrode lead, a first working electrode lead, a second working electrode lead, a counter electrode terminal, and a working electrode terminal, respectively.

### Example 1

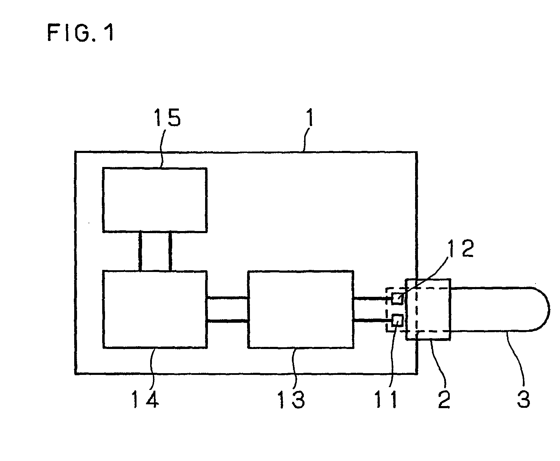

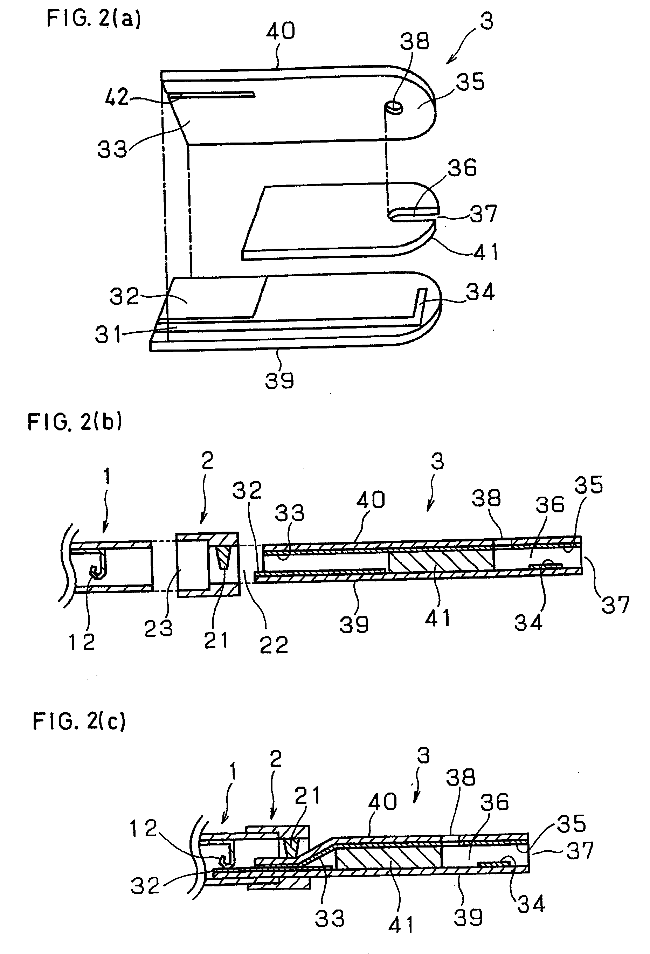

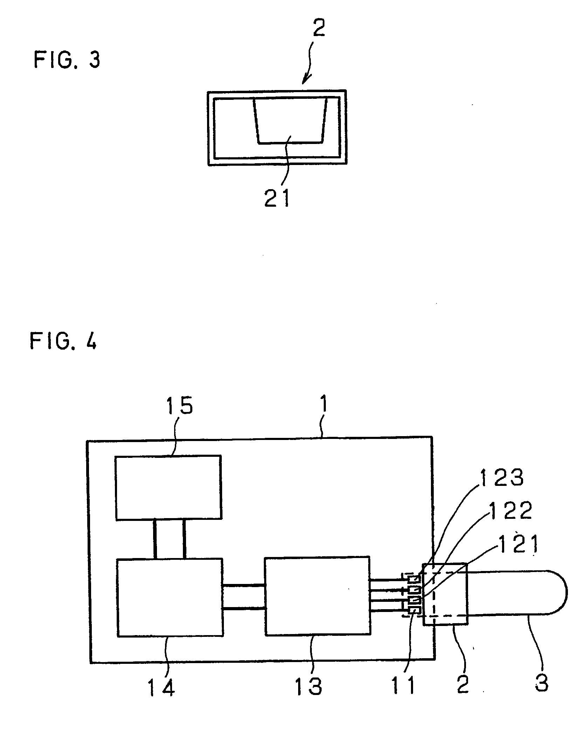

Example 1 of the present invention is illustrated in FIGS. 1 to 3. FIG. 1 is a schematic view showing the constitution of a biosensor, an adapter, and a measuring device of this embodiment. FIG. 2 is schematic views showing the constitution of the biosensor, the adapter, and the vicinity of the sensor slot of the measuring device of this embodiment. FIG. 2(a) is an exploded perspective view of the biosensor of this embodiment, except for the reagent layer and the surfactant layer of the biosensor. FIG. 2(b) is a longitudinal sectional view of this embodiment showing the sensor slot of the measuring device, the adapter, and the biosensor separated from one another. FIG. 2(c) is a longitudinal sectional view of this embodiment showing the sensor slot of the measuring device, the adapter, and the biosensor connected to one another. FIG. 3 is a front view seen from the direction of the sensor slot of the adapter.

In FIG. 1, a measuring device 1 comprises a working electrode terminal 11, a counter electrode terminal 12, a measuring part 13, a computing part 14, and a data display part 15, and has the same constitution as that of the measuring device for the conventional flat-type biosensors.

A biosensor 3 is detachably connected to the measuring device 1 through an adapter 2. In FIG. 2(a), the biosensor 3 comprises a working electrode base plate 39, a counter electrode base plate 40, a working electrode lead 31, a first counter electrode lead 32, a second counter electrode lead 33, a working electrode 34, a counter electrode 35, a reagent layer (not shown), a spacer member 41 having a slit to form a sample feed portion 36, an air hole 38 as an opening communicating with the sample feed portion 36, and a sample feed port 37.

A palladium thin film was deposited on the working electrode base plate 39 by sputtering and then patterned by photolithography and dry etching to form the working electrode lead 31, the first counter electrode lead 32, and the working electrode 34. In the same manner, a palladium thin film was deposited on the whole surface of the counter electrode base plate 40 by sputtering to form the second counter electrode lead 33 and the counter electrode 35. The length of the counter electrode base plate 40 in the longitudinal direction is shorter than that of the working electrode base plate 39. The counter electrode base plate 40 is provided with a slit 42 to define the width of the end portion of the second counter electrode lead 33 as being equal to or shorter than the width of the first counter electrode lead 32.

An aqueous solution containing GOD as an oxidation-reduction enzyme and potassium ferricyanide as an electron mediator was dropped on the working electrode 34 followed by drying to form a reagent layer. For the purpose of carrying out sample feed smoothly, a surfactant layer containing lecithin as a surfactant was formed on the reagent layer and on the working electrode base plate 39 facing to the sample feed portion 36. Finally, the working electrode base plate 39, the spacer member 41, and the counter electrode base plate 40 were bonded to produce the biosensor 3. In biosensor 3, a part of the palladium film on the working electrode base plate 39, which is exposed to the sample feed portion 36, functions as the working electrode 34. A part of the palladium film on the counter electrode base plate 40, which is exposed to the sample feed portion 36, functions as the counter electrode 35.

The adapter 2 has a sensor port 22 into which a part of the biosensor 3 is inserted from the direction of the lead formed portion. The adapter further includes a connection port 23 for connection to the measuring device and a pressing portion 21 for pressing at least a part of the counter electrode base plate 40 of the biosensor 3.

For measurement, the connection port 23 of the adapter 2 is attached to the measuring device 1, and the biosensor 3 is attached from the sensor port 22 of the adapter 2. Thus, a part of the counter electrode base plate 40 is pressed by the pressing portion 21, so that the end portion of the counter electrode base plate 40 is bent to bring the first counter electrode lead 32 and the second counter electrode lead 33 into contact with each other. At this time, since the length of the counter electrode base plate 40 in the longitudinal direction is shorter than that of the working electrode base plate 39, the end portions of the working electrode lead 31 and the first counter electrode lead 32, both of which are formed on the working electrode base plate 39, are exposed to the outside of the sensor. The exposed portions of the working electrode lead 31 and the first counter electrode lead 32 serve as the connecting terminals on the sensor side and connect to the working electrode terminal 11 and the counter electrode terminal 12, respectively, on the side of measuring device 1 (FIG. 2(c)). When a voltage is applied between the working electrode terminal 11 and the counter electrode terminal 12, it is possible to apply a voltage between the working electrode 34 and the counter electrode 35.

An aqueous β-D-glucose solution was used as a sample solution. The concentration of β-D-glucose in the sample solution was determined. Several kinds of sample solutions having different concentrations of β-D-glucose were prepared. Each sample solution was brought into contact with the sample feed port 37 of the biosensor 3. Since the air hole 38 was communicating with the sample feed port 37, the sample solution introduced into the sample feed port 37 was permeated to inside by capillary phenomenon and thus fed to the sample feed portion 36. A voltage of 300 mV based on the counter electrode 35 was applied to the working electrode 34 by the measuring portion 13 of the measuring device 1, at which time the value of current flowing through the working electrode 34 was measured with the measuring part 13. The value obtained with the measuring part 13 was converted into a concentration by reference to the prestored calibration curve in the computing part 14, and the result thus obtained was displayed on the data display part 15.

### Example 2

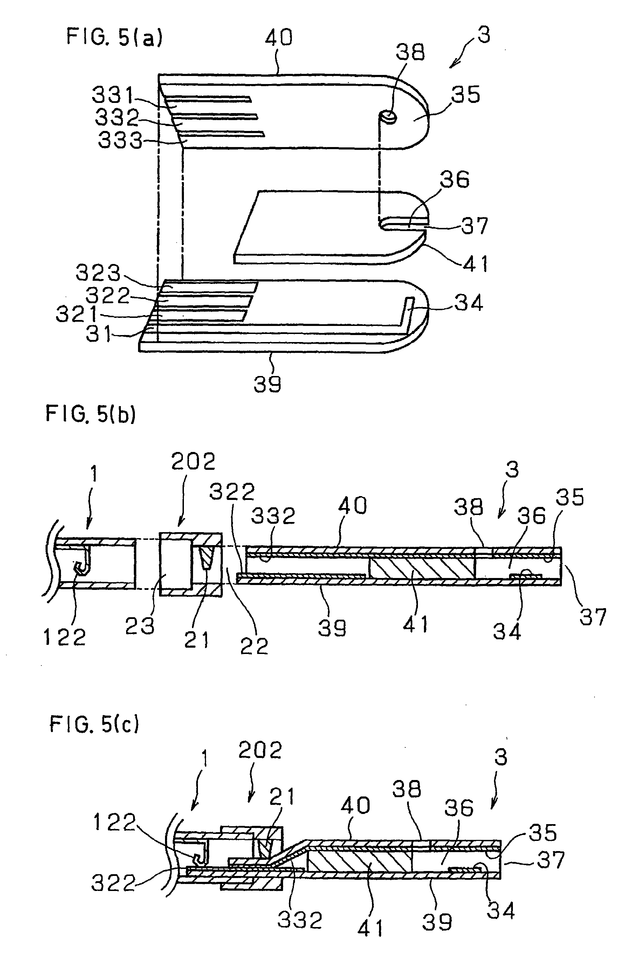

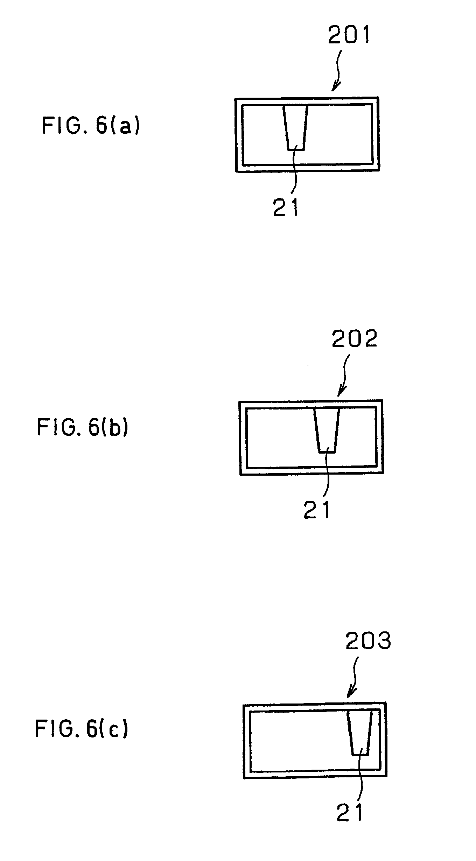

Example 2 of the present invention is illustrated in FIGS. 4 to 6. This embodiment is different from Example 1 in that the first and second counter electrode leads of the biosensor is divided into three leads and the measuring device is provided with three counter electrode terminals. FIG. 4 is a schematic view of this embodiment showing the constitution of a biosensor, an adapter, and a measuring device. FIG. 5 is schematic views of this embodiment showing the sensor, the adapter, and the vicinity of the sensor slot of the measuring device. FIG. 5(a) an exploded perspective view of the biosensor of this embodiment except for the reagent layer and the surfactant layer of the biosensor. FTG. 5(b) is a longitudinal sectional view of this embodiment showing the sensor slot of the measuring device, the adapter, and the biosensor are separated from one another. FIG. 5(c) is a longitudinal sectional view of this embodiment showing how the sensor slot of the measuring device, the adapter, and the biosensor are connected to one another. FIGs. 6(a), 6(b) and 6(c) are front views seen from the direction of the sensor slots of three kinds of adapters with pressing portions at different positions.

In FIG. 4, a measuring device 1 comprises a working electrode terminal 11, a counter electrode terminal 121, counter electrode terminal 122, a counter electrode terminal 123, a measuring part 13, a computing part 14, and a data display part 15.

Similarly to Example 1, the biosensor 3 is detachably connected to the measuring device 1 through an adapter 2. The biosensor 3 comprises a working electrode base plate 39, a counter electrode base plate 40, a working electrode lead 31, a first counter electrode lead 321, a first counter electrode lead 322, a first counter electrode lead 323, a second counter electrode lead 331, a second counter electrode lead 332, a second counter electrode lead 333, a working electrode 34, a counter electrode 35, a reagent layer (not shown), a spacer member 41 having a slit to form a sample feed portion 36, an air hole 38 as an opening communicating with the sample feed portion 36, and a sample feed port 37.

The biosensor 3 was produced in the same manner as Example 1, except that three first counter electrode leads 321, 322, and 323 were provided on the counter electrode base plate 39. Also, three slits are provided on the counter electrode base plate 40 on the counter electrode base plate 40 so that three second counter electrode leads 331, 332, and 333 were formed at the positions opposite the first counter electrode leads 321, 322 and 323 on the counter electrode base plate 40. Adapters 201, 202, and 203 in this embodiment as shown in FIGS. 6(a), 6(b) and 6(c), respectively, have a sensor port 22, a connection port 23, and a pressing portion 21 similar to Example 1. However, the width of the pressing portion 21 is narrower than that of Example 1.

In the production process for the biosensor 3, for example, lot numbers A, B, and C at three ranks have been given, depending upon the performance from the results of characteristic inspection. When the lot number of the biosensor 3 to be used for measurement is A, the adapter 201 shown in FIG. 6(a) is used. The adapter 201 is provided with the pressing portion 21 on the leftmost side seen from the direction of the sensor slot 22, and when the adapter 201 is attached to the measuring device 1 and the biosensor 3 is inserted into the adapter 201, a part of the counter electrode base plate 40 is pressed by the pressing portion 21, so that the end potion of the counter electrode base plate 40 is bent and the first counter electrode lead 321 is brought into contact with the second counter electrode lead 331. When the lot number of the biosensor 3 to be used for measurement is B, the adapter 202 shown in FIG. 6(b) is used. The adapter 202 is provided with the pressing portion 21 at the center seen from the direction of the sensor port 22, and when the adapter 202 is attached to the measuring device 1 and the biosensor 3 is inserted into the adapter 202, the first counter electrode lead 322 is brought into contact with the second counter electrode lead 332. Similarly, when the lot number of the biosensor 3 to be used for measurement is C, the adapter 203 shown in FIG. 6(c) is used. The adapter 203 is provided with the pressing portion 21 on the rightmost side seen from the direction of the sensor slot 22, and when adapter 203 is attached to the measuring device 1 and the biosensor 3 is inserted into the adapter 203, a part of the counter electrode base plate 40 is pressed by the pressing portion 21, so that the end potion of the counter electrode base plate 40 is bent and the first counter electrode lead 323 is brought into contact with the second counter electrode lead 333.

An aqueous β-D-glucose solution was used as a sample solution. The concentration of β-D-glucose in the sample solution was determined in the same manner as Example 1. The sample solution was brought into contact with the sample feed port 37 of the biosensor 3, so that the sample solution was introduced into the sample feed portion 36. After a predetermined period of time, under the condition that the sample solution was in contact with the working electrode 34 and the counter electrode 35, the values of resistance or the like, between the working electrode terminal 11 and the counter electrode terminal a 121, between the working electrode terminal 11 and the counter electrode terminal 122, and between the working electrode terminal 11 and the counter electrode terminal 123 were measured in the measuring device 1 to detect which of three second counter electrode leads 331, 332, and 333 was connecting with the first counter electrode lead on the side of the measuring device 1. Thus, the user is able to determine in the measuring device 1, which of A, B, and C was the lot number of the sensor.

A voltage of 300 mV based on the counter electrode 35 was applied to the working electrode 34 by the measuring part 13 of the measuring device 1, at which time the value of current flowing through the working electrode 34 was measured with the measuring part 13. Depending upon the lot number determined, the calibration curve corresponding to the lot number, which calibration curve had been prestored in the measuring device 1, was automatically selected, and the value of current obtained in the measuring part 13 was converted into a concentration by reference to the selected calibration curve in the computing part 14. The result thus obtained was displayed on the data display part 15. Therefore, in this embodiment, the correction depending upon the production lot of a biosensor is carried out only by the selection of an adapter to be used, so that the accurate determination of a substrate can easily be achieved.

In Example 2, the second counter electrode lead is divided into three separate leads. However, the present invention is not limited thereto, and the same advantageous effects can be obtained, so long as the second counter electrode lead is divided into two or more leads. The greater number of divided leads is preferred because the correction of measured values in accordance with differences in the production lots of biosensors can be carried out in detail.

Example 2 describes the case where there is a single contact point between the plurality of the first counter electrode leads and the plurality of the second counter electrode leads. However, the present invention is not limited thereto. For example, a plurality of the counter leads can be contacted at a plurality of points. If an adaptor (not shown) capable of contacting the first counter electrode leads 321 and 322 with the second counter electrode leads 331 and 332, respectively, as employed in the above Example 2, a new lot number D of the biosensor can be determined.

The above Example 2 describes an example where the production lot of the biosensor is determined, but the present invention is not limited thereto. For example, a target to be measured by the biosensor can be determined. In such a case, it is possible to measure three types of biosensors using only one measuring device where the substrates as the targets to be measured are glucose, lactic acid and cholesterol, respectively. In this case, calibration curves stored in the measuring device 1 are based not on the lot number, but on the substrate. Further, the number of the divisions of the first counter electrode lead is not limited to three as in the case of the determination of the production lot number, but can be more or less than three.

### Example 3

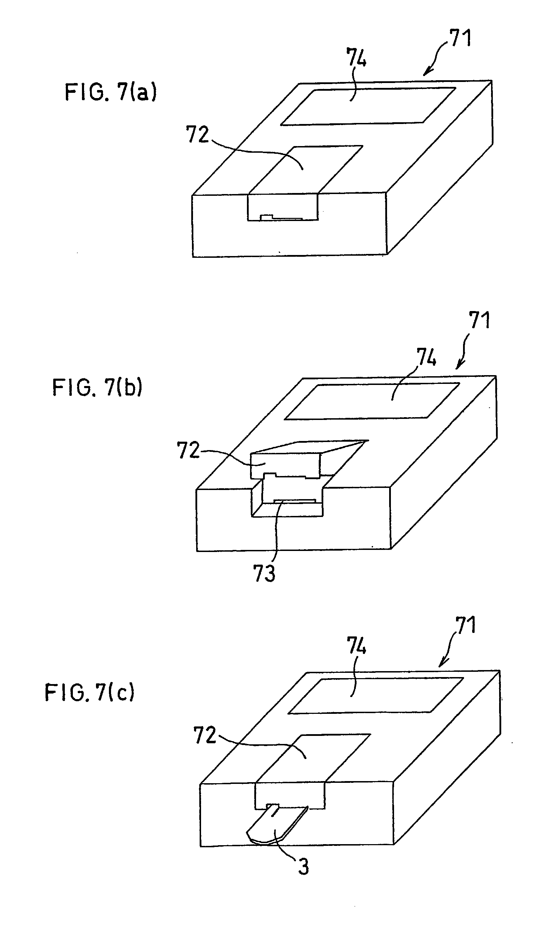

Example 3 of the present invention is illustrated in FIG. 7. FIG. 7 is a schematic view showing a biosensor and a measuring device of this embodiment. FIG. 7(a) is a schematic view showing the measuring device wherein the pressing portion is closed. FIG. 7(b) is a schematic view showing the measuring device wherein the pressing portion is open. FIG. 7(c) is a schematic view showing the measuring device wherein the biosensor is connected to the measuring device. In this embodiment, the same biosensor as that in Example 1 was used. The procedure of the measurement is described below.

A pressing portion 72 of the measuring device is opened and a part of a biosensor 3 is inserted into the sensor port 73. When the biosensor 3 is inserted, a working electrode lead 31 and a first counter electrode lead 32 of the biosensor 3 are connected to a working electrode terminal (not shown) and a counter electrode terminal (not shown) of the measuring device 71. The working electrode terminal and the counter electrode terminal are located at the vicinity of the sensor port 73 in the measuring device 71.

The opened pressing portion 72 is closed by pressing a part of a counter electrode base plate 40 of the biosensor 3 using pressing portion 72, so that the end portion of the counter electrode base plate 40 is bent to bring the first counter electrode lead 32 and the second counter electrode lead 33 into contact with each other. When a voltage is applied between the working electrode terminal and the counter electrode terminal, it is possible to apply a voltage between the working electrode 34 and the counter electrode 35.

An aqueous β-D-glucosc solution was used as a sample solution. The concentration of β-D-glucose in the sample solution was determined in the same manner as in Example 1. Thus, the substrate contained in the sample solution can be determined by connecting the biosensor of the present invention directly to the measuring device without using the adaptor.

It is noted that, in Example 3, the measuring device can be constituted such that a part of the working electrode terminal or the counter electrode terminal can be used as the pressing portion. Thus, the number of members constituting the measuring device can be decreased.

The voltage applied to the working electrode 34 was 300 mV based on the counter electrode 35 in the above embodiments. However, the voltage is not limited thereto, but it may be a voltage such that an electron mediator can cause electrode reaction on the working electrode 34.

The position of the air hole 38 is not limited to that shown in FIGS. 2(a) and 5(a), but it may be positioned anywhere if it communicates with the sample feed portion 36 and is on the opposite side of the sample feed port 37 against the sample feed portion 36.

Further with respect to the above embodiments, a reagent layer is formed by applying a solution containing an oxidation-reduction enzyme and then drying. However, the present invention is not limited thereto. For example, a solution containing a reagent may be applied by the ink jet system. Thus, even if the amount of a solution to be applied is minute, the accurate position control of a reagent layer can be achieved. Alternatively, a glass filter paper, which is allowed to carry a solution containing a reagent and then dried, may be positioned in the sample feed portion 36. Further, an electrode may be formed by mixing an electrically conductive material and a reagent. The position to carry a reagent may preferably be on the working electrode 34 or the counter electrode 35. However, the position is not limited thereto, but may be any position other than on the working electrode 34 and the counter electrode 35 in the sample feed portion 36, so long as it may be a position at which the reagent can be brought into contact with a sample.

As for the spacer member 41, any material can be used for the spacer member, so long as it is a material having electrically insulating properties and stiffness sufficient for storage and measurement. For example, the material can be thermoplastic resins such as polyethylene, polystyrene, polyvinyl chloride, polyamide, and saturated: polyester resins; and thermosetting resins such as urea resins, melamine resins, phenol resins, epoxy resins, and unsaturated polyester resins.

For the working electrode, any material can be used, so long as it is an electrically conductive material which itself cannot be oxidized when oxidizing an electron mediator. For the counter electrode, any material can be used, so long as it is an electrically conductive material. For example, the material may be palladium, silver, platinum, and carbon. Alternatively, the surface of an electrically insulating material may be coated with any of these electrically conductive materials.

In the above embodiments, the electrodes can be formed by sputtering, followed by photolithography and etching. However, the present invention is not limited thereto. For example, other methods may be used such as by sputtering a noble metal such as palladium onto a base plate followed by laser trimming or by screen printing an electrically conductive paste onto a base plate.

Further, in the above embodiments, the second base plate was shorter in the longitudinal direction than the first base plate. However, the present invention is not limited thereto. The sizes and arrangement of the first base plate and the second base plate may be adjusted so that at least end portions of the first lead and the second lead are exposed to outside when at least a part of the second base plate is pressed to bring the second lead and the third lead into electrical contact with each other.

For the enzyme, it may include, for example, fructose dehydrogenase, glucose oxidase, glucose dehydrogenase, alcohol oxidase, lactate oxidase, cholesterol oxidase, xanthin oxidase, and amino acid oxidase.

The electron mediator may be potassium ferricyanide, p-benzoquinone, phenazine methosulfate, methylene blue, ferrocene derivatives, or mixtures thereof. When oxygen is used as an electron mediator, a current response can also be obtained.

In the above embodiments, an aqueous β-D-glucose solution is used as a sample. However, the present invention is not limited thereto. For example, the present invention can also be applied to biological samples such as whole blood, plasma, serum, interstitial fluid, sputum, and urine. As the whole blood, the invention can be used for capillary blood collected by pricking the skin of a fingertip or an arm, or for venous blood, arterial blood, and the like.

As described above, the present invention makes it possible to provide a face-type biosensor which has simple leads and can easily be connected to a measuring device through an adaptor or a pressing portion. Also, the embodiments described disclose the first base plate as including the working electrode and the second base plate as including the counter electrode, it is within the scope of the invention that the first base plate can function as the counter electrode while the second base place can function as the counter electrode while the second base place can function as the working electrode.

Although the present invention has been described in terms of the presently preferred embodiments, it is to be understood that such disclosure is not to be-interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

## Claims

1. A biosensor having a first base plate and a second base plate with a sample feed portion disposed therebetween, said biosensor comprising:
a first electrode, a first lead, and a second lead formed on said first base plate and on the side of said first base plate facing said second base plate; and
a second electrode and a third lead formed on said second base plate and on the side of said second base plate facing said first base plate;
wherein either said first electrode or said second electrode functions as a working electrode while the other electrode functions as a counter electrode; and said second lead and said third lead are brought into electrical contact with each other by pressing said first base plate and/or said second base plate toward the other base plate.

2. The biosensor in accordance with claim 1, wherein said third lead is divided into more than one portion and said biosensor includes more than one second lead.

3. The biosensor in accordance with claim 1 or claim 2, wherein a spacer member is provided between said first base plate and said second base plate.

4. The biosensor in accordance with claim 3, wherein the spacer is attached to at least one base plate of said first base plate and said second base plate.

5. The biosensor in accordance with claim 4, wherein the spacer is attached to the first base plate.

6. The biosensor in accordance with claim 4, wherein the spacer is attached to the second base plate.

7. The biosensor in accordance with any preceding claim, wherein the length of the second base plate in the longitudinal direction is shorter than that of the first base plate.

8. The biosensor in accordance with any preceding claim, wherein the sample feed portion includes a substrate having a reagent disposed thereon containing an enzyme selected from the group consisting of fructose dehydrogenase, glucose oxidase, glucose dehydrogenase, alcohol oxidase, lactate oxidase, cholesterol oxidase, xanthin oxidase, and amino acid oxidase.

9. An adapter comprising a connection port for connecting to a measuring device, a sensor slot into which a biosensor is inserted, and a pressing portion.

10. The adaptor of claim 9, wherein the adapter receives said biosensor inserted into said sensor slot, the biosensor comprising a first base plate and a second base plate with a sample feed portion therebetween, a first electrode, a first lead, and a second lead formed on said first base plate and on the side of said first base plate facing said second base plate; and a second electrode and a third lead formed on said second base plate and on the side of said second base plate facing said first base plate; wherein said second lead and said third lead are brought into electrical contact with each other by pressing said pressing portion, said pressing portion causes at least a part of said first base plate or said second base plate to move toward the other base plate.

11. The adapter in accordance with claim 10, wherein said third lead of said biosensor is divided into more than one portion and said biosensor includes more than one second lead.

12. The adapter in accordance with claim 10, wherein a spacer member is provided between said first base plate and said second base plate of said biosensor.

13. The adapter in accordance with claim 12, wherein the spacer of said biosensor is attached to at least one of said first base plate or said second base plate.

14. The adapter in accordance with claim 13, wherein the spacer of said biosensor is attached to the first base plate.

15. The adapter in accordance with claim 13, wherein the spacer of said biosensor is attached to the second base plate.

16. The adaptor in accordance with any one of claims 10 to 15, wherein the length of the second base plate in the longitudinal direction is shorter than that of the first base plate.

17. The adaptor in accordance with any one of claims 9 to 16, wherein said pressing portion comprises an elastic material.

18. A measuring device comprising a biosensor, an adapter including a pressing portion, and a measuring means.

19. The measuring device of claim 18, wherein the biosensor comprises a first base plate and a second base plate with a sample feed portion disposed therebetween, a first electrode, a first lead, and a second lead formed on said first base plate and on the side of said first base plate facing said second base plate; and a second electrode and a third lead formed on said second base plate and on the side of said second base plate facing said first base plate; wherein said second lead and said third lead are brought into electrical contact with each other by pressing said pressing portion, said pressing portion causing at least a part of said first base plate or second base plate to move toward the other base plate.

20. The measuring device in accordance with claim 19, wherein said third lead of said biosensor is divided into more than one portion and said biosensor includes more than one second lead.

21. The measuring device in accordance with claim 19, wherein a spacer member is provided between said first base plate and said second base plate of said biosensor.

22. The measuring device in accordance with claim 21, wherein the spacer of said biosensor is attached to at least one of said first base plate and said second base plate.

23. The measuring device in accordance with claim 22, wherein the spacer of said biosensor is attached to the first base plate.

24. The measuring device in accordance with claim 22, wherein the spacer of said biosensor is attached to the second base plate.

25. The measuring device in accordance with any one of claims 19 to 24, wherein the length of the second base plate in the longitudinal direction is shorter than that of the first base plate.

26. The measuring device in accordance with any one of claims 19 to 25, wherein said pressing portion comprises an elastic material.

27. The measuring device in accordance with any one of claims 19 to 26, wherein the sample feed portion includes a reagent containing an enzyme selected from the group consisting of fructose dehydrogenase, glucose oxidase, glucose dehydrogenase, alcohol oxidase, lactate oxidase, cholesterol oxidase, xanthin oxidase, and amino acid oxidase.

28. The measuring device of any one of claims 19 to 27, wherein said measuring device measures a substrate contained in a sample solution.

29. The measuring device of any one of claims 19 to 28, wherein the measuring device includes a first terminal electrically connected to said first electrode of said biosensor, and at least one second terminal electrically connected respectively to said second electrode of said biosensor.

30. The measuring device of any one of claims 19 to 29, wherein said measuring means comprises a means of applying a voltage between said first electrode and said second electrode of said biosensor through said first terminal and said at least one second terminal, and a means of measuring an electrical change between said first electrode and said second electrode.

31. The measuring device in accordance with claim 29, wherein said measuring device comprises a plurality of second terminals.

32. The measuring device in accordance with claim 31, wherein said measuring device further includes means for measuring an electrical change between said first terminal and said plurality of second terminals.

33. The measuring device in accordance with claim 31, wherein said measuring device further includes means for sensing a second terminal electrochemically connected to said first terminal out of said plurality of said second terminals, means for determining a type of biosensor on the basis of a number and a position of the sensed second terminal, and means for correcting a measurement result according to the type of biosensor.

34. A measuring device comprising:
a pressing portion;
a sensor port for receiving a biosensor, said biosensor having a first lead, a second lead, and a third lead, said first lead electrically connected to a first electrode and said third lead electrically connnected to a second electrode;
a first terminal for electrically connecting said first lead of said biosensor to said measuring device
a second terminal for electrically connecting said second lead of said biosensor to said measuring device;
a means for applying a voltage between said first electrode and said second electrode; and
a means for measuring an electrical change between said first electrode and said second electrode.

35. The measuring device in accordance with claim 34, wherein said measuring device comprises comprises a plurality of second terminals.

36. The measuring device in accordance with claim 35, wherein said measuring device includes a means for measuring an electrical change between said first terminal and said plurality of said second terminals.

37. The measuring device in accordance with any one of claims 34 to 36, wherein said pressing portion comprises an elastic material.

38. The measuring device in accordance with any one of claims 34 to 37, wherein said second terminal serves also as said pressing portion.

39. The measuring device in accordance with any one of claims 34 to 38, wherein the biosensor comprises a first base plate and a second base plate with a sample feed portion therebetween, said first electrode, said first lead, and said second lead formed on said first base plate and on the side of said first base plate facing said second base plate; and said second electrode and said third lead formed on said second base plate and on the side of said second base plate facing said first base plate; wherein said second lead and said third lead are brought into electrical contact with each other by pressing said pressing portion, said pressing portion causing at least a part of said first base plate or said second base plate to more toward the other base plate.

40. The measuring device in accordance with claim 39, wherein said third lead of said biosensor is divided into more than one portion and said biosensor includes more than one second lead.

41. The measuring device in accordance with claim 39, wherein a spacer member is provided between said first base plate and said second base plate of said biosensor.

42. The measuring device in accordance with claim 41, wherein the spacer of said biosensor is attached to at least one of said first and said second base plate.

43. The measuring device in accordance with claim 42, wherein the spacer of said biosensor is attached to the first base plate.

44. The measuring device in accordance with claim 42, wherein the spacer of said biosensor is attached to the second base plate.

45. The measuring device in accordance with any one of claims 39 to 44, wherein the length of the second base plate in the longitudinal direction is shorter than that of the first base plate.

46. The measuring device in accordance with any one of claims 39 to 45, wherein the sample feed portion includes a reagent containing an enzyme selected from the group consisting of fructose dehydrogenase, glucose oxidase, glucose dehydrogenase, alcohol oxidase, lactate oxidase, cholesterol oxidase, xanthin oxidase, and amino acid oxidase.

47. The measuring device of any one of claims 39 to 46, wherein said measuring device measures a substrate contained in a sample solution.

48. The measuring device according to claim 47, wherein said device measures the substrate contained in a sample solution fed into the sample feed portion of said biosensor, said pressing portion pressing at least a part of said second base plate of said biosensor to bring said second lead and said third lead into electrical contact with each other.

49. The measuring device in accordance with claim 35, wherein said measuring device includes means for sensing a second terminal electrochemically connected to said first terminal out of said plurality of said second terminal, means of determining a type of biosensor on the basis of a number and a position of the sensed second terminal, and means for correcting a measurement result according to the type of biosensor.

50. The measuring device in accordance with claim 39, wherein said pressing portion is movable so that a position to be pressed of said second base plate of said biosensor is determined by said pressing portion.
